# EUROPÄISCHE PATENTANMELDUNG

(11) **EP 4 537 808 A1**
(43) Veröffentlichungstag der Anmeldung: **16.04.2025**
(21) Anmeldenummer: 23202965.2
(22) Anmeldetag: 11.10.2023
(51) Int. Cl.: A61K 8/65, A61K 8/92, A61K 8/9789, A61K 8/9794, A61K 36/23, A61K 36/28, A61K 38/39, A61P 17/00, A61Q 19/00, A61Q 19/02, A61Q 19/08

(54) **HAUTPFLEGEZUSAMMENSETZUNG**

(71) Anmelder: Bulut, Evrim, 56203 Höhr-Grenzhausen (DE)
(72) Erfinder: Bulut, Evrim, 56203 Höhr-Grenzhausen (DE)
(74) Vertreter: dompatent von Kreisler Selting Werner - Partnerschaft von Patent- und Rechtsanwälten mbB

(57) **Zusammenfassung**

Hautpflegezusammensetzung umfassend
(i) Collagen,
(ii) eine aus Strohblumen (*Helichrysum*) erhältliche Komponente,
(iii) eine aus Schwarzkümmel (*Nigella*) erhältliche Komponente, und
(iv) eine aus Disteln (*Carduoideae*) erhältliche Komponente.

## Beschreibung

Die vorliegende Erfindung betrifft eine Hautpflegezusammensetzung, insbesondere zur kosmetischen Verwendung, sowie ein Verfahren zu deren Herstellung.

Hautpflegezusammensetzungen, auch als "Hautcreme" oder "Hautcremes" bezeichnet, dienen dem Schutz, der Pflege, der Verschönerung und der Korrektur der (menschlichen) Haut. Eine wichtige Forderung an Hautpflegezusammensetzungen oder Hautcremes ist zum einen eine gute Hautverträglichkeit. Um dieses Ziel zu erreichen, sind Formulierungen notwendig, die den natürlichen Funktionen und Mechanismen der Haut weitgehend entsprechen und die aufgrund ihres Aufbaus keine problematischen Reaktionen von Substanzen untereinander erwarten lassen. Eine weitere wichtige Forderung an eine Hautpflegezusammensetzung ist die Förderung der Verbesserung der Hautstruktur. Insbesondere besteht der Wunsch, die Bildung von (altersbedingten) Hautfalten zu verhindern bzw. hinauszuzögern, sowie bereits vorhandene Falten zu entfernen oder abzuschwächen. Zudem besteht Interesse daran, das Auftreten von Pigmentstörungen, Sommersprossen oder Altersflecken zu verringern, die Haut mit Feuchtigkeit zu versorgen und das Hautbild allgemein zu verbessern.

Bei der Hautalterung wird zwischen der biologischen Hautalterung und der Alterung durch äußere Einflüsse unterschieden. Sichtbares äußeres Zeichen des Alterns der Haut, das etwa ab dem 25. Lebensjahr auftritt, ist das Entstehen von sichtbaren Falten. Bei der biologischen Hautalterung verlangsamt sich die Geschwindigkeit der Zellerneuerung, die Fähigkeit der Haut, Feuchtigkeit zu speichern, nimmt ab. In tieferen Hautschichten wird mit zunehmendem Alter immer mehr Collagen abgebaut, wodurch die Haut an Elastizität verliert und sich besagte Falten bilden.

Da deutlich sichtbare Falten als Zeichen des Älterwerdens zumindest im westlichen Kulturkreis nicht als erstrebenswert gelten, werden zahlreiche Kosmetikprodukte, insbesondere in Form von Hautcremes angeboten, die zur Faltenreduktion geeignet sein sollen. Derartige handelsübliche Produkte weisen in der Regel verschiedene Inhaltsstoffe wie Vitamin C, Retinol, Coenzym Q10, Hyaluronsäure, aber auch Kaviarextrakt und sogar Goldpartikel auf.

Von Nachteil ist, dass der Wirkungsgrad dieser bekannten Hautpflegeprodukte häufig unbefriedigend gering ist. Außerdem weisen einige der bekannten Produkte eine unzureichende Hautverträglichkeit auf und sind beispielsweise nicht für Menschen mit empfindlicher oder zu allergischen Reaktionen neigender Haut geeignet.

Es besteht somit Bedarf an weiteren Hautpflegezusammensetzungen, insbesondere zur Reduktion von (altersbedingten) Hautfalten.

Es ist daher Aufgabe der vorliegenden Erfindung, eine Hautpflegezusammensetzung sowie ein Verfahren zu deren Herstellung zur Verfügung zu stellen, welche zumindest einige der aus dem Stand der Technik bekannten Probleme überwinden.

Gelöst wird die erfindungsgemäße Aufgabe durch eine Hautpflegezusammensetzung umfassend
(i) Collagen,
(ii) eine aus Strohblumen (*Helichrysum*) erhältliche Komponente,
(iii) eine aus Schwarzkümmel (*Nigella*) erhältliche Komponente, und
(iv) eine aus Disteln (*Carduoideae*) erhältliche Komponente.

Die erfindungsgemäße Hautpflegezusammensetzung ist dazu geeignet, auf die Haut aufgetragen zu werden. Bei der Hautpflegezusammensetzung kann es sich um eine Salbe, eine Creme, ein Öl, eine Lotion, ein Serum oder ein Gel handeln. Bevorzugt handelt es sich um eine Salbe, eine Creme, ein Serum oder ein Öl.

Die erfindungsgemäße Hautpflegezusammensetzung umfasst als eine Komponente Collagen. Collagen ist ein bio-degradierbares, biokompatibles Protein, welches in der medizinischen Anwendung breite Verwendung findet, beispielsweise als Wundheilungsmittel, Trägermaterial in Wundauflagen oder als Hämostyptikum. Auch in der Kosmetik ist die Verwendung von Collagen, beispielsweise als feuchtigkeitsregulierender Wirk- oder Hilfsstoff oder als Trägermaterial für kosmetische Auflagen verbreitet. Bevorzugt ist das in der erfindungsgemäßen Hautpflegezusammensetzung verwendete Collagen aus der Gruppe der tierischen Collagene ausgewählt. Das Collagen der vorliegenden Erfindung kann nach üblichen Verfahren aus den üblichen Quellen wie Knochen, Häuten oder Sehnen gewonnen werden. Verfahren zur Gewinnung von Collagen aus Knochen, Häuten oder Sehnen sind dem Fachmann bekannt. Bevorzugt wird das Collagen aus den Knochen, Häuten oder Sehnen von Schafen, Ziegen, Schweinen oder Rindern gewonnen. In einer besonders bevorzugten Ausführungsform der vorliegenden Erfindung handelt es sich bei dem Collagen um Collagen, welches aus den Knochen von Schafen gewonnen wurde.

Die erfindungsgemäße Hautpflegezusammensetzung umfasst zudem eine aus Strohblumen (*Helichrysum*) erhältliche Komponente. Die Pflanzengattung der Strohblumen (*Helichrysum*) gehört zur Familie der Korbblütler (*Asteraceae*)*.* Die etwa 600 Arten sind hauptsächlich in der Alten Welt, besonders im Südlichen Afrika und Madagaskar beheimatet.

Bei der aus Strohblumen (*Helichrysum*) erhältlichen Komponente der vorliegenden Erfindung kann es sich um einen Extrakt aus Strohblumen (*Helichrysum*) oder Pflanzenteilen von Strohblumen (*Helichrysum*) oder um ein Strohblumen (*Helichrysum*)-Öl handeln. Strohblumen *(Helichrysum)*-Öl ist ein aus Strohblumen (*Helichrysum*) oder Teilen davon gewonnenes Öl. Das Öl kann sowohl aus frischen als auch aus getrockneten Strohblumen oder Teilen davon gewonnen werden. Verfahren zur Herstellung von Ölen aus Pflanzen oder Pflanzenteilen sind dem Fachmann bekannt. Bei dem Extrakt kann es sich um einen anorganischen oder organischen Extrakt handeln, es können als Extraktionsmittel also sowohl anorganische Lösungsmittel, wie Wasser, Säuren oder Basen, als auch organische Lösungsmittel, wie Alkohole, Terpene, Diethylether, pflanzliche Öle, chlorierte Kohlenwasserstoffe oder n-Hexan, eingesetzt werden. Bevorzugt handelt es sich um einen organischen Extrakt, mehr bevorzugt um einen organischen Extrakt mit einem pflanzlichen Öl als Extraktionsmittel. Bevorzugt handelt es sich um einen organischen Extrakt mit Olivenöl als Extraktionsmittel.

Die erfindungsgemäße Hautpflegezusammensetzung umfasst zudem eine aus Schwarzkümmel (*Nigella*) erhältliche Komponente. Die Schwarzkümmel (*Nigella*) sind eine Pflanzengattung innerhalb der Familie der Hahnenfußgewächse (*Ranunculaceae*)*.*

Bei der aus Schwarzkümmel (*Nigella*) erhältlichen Komponente der vorliegenden Erfindung kann es sich um einen Extrakt aus Schwarzkümmel (*Nigella*) oder Pflanzenteilen von Schwarzkümmel (*Nigella*) oder um ein Schwarzkümmel (*Nigella*)-Öl handeln. Schwarzkümmel (*Nigella*)-Öl ist ein aus Schwarzkümmel (*Nigella*) oder Teilen davon gewonnenes Öl. Verfahren zur Herstellung von Ölen aus Pflanzen oder Pflanzenteilen sind dem Fachmann bekannt. Das Öl kann sowohl aus frischen als auch aus getrockneten Pflanzen oder Pflanzenteilen gewonnen werden. Bei dem Extrakt kann es sich um einen anorganischen oder organischen Extrakt handeln, es können als Extraktionsmittel also sowohl anorganische Lösungsmittel, wie Wasser, Säuren oder Basen, als auch organische Lösungsmittel, wie Alkohole, Terpene, Diethylether, pflanzliche Öle, chlorierte Kohlenwasserstoffe oder n-Hexan, eingesetzt werden. Bevorzugt handelt es sich um einen organischen Extrakt, mehr bevorzugt um einen organischen Extrakt mit einem pflanzlichen Öl als Extraktionsmittel. Bevorzugt handelt es sich um einen organischen Extrakt mit Olivenöl als Extraktionsmittel.

Die erfindungsgemäße Hautpflegezusammensetzung umfasst zudem eine aus Disteln (*Carduoideae*) erhältliche Komponente. "Disteln" im Sinne dieser Erfindung sind Pflanzen der *Carduoideae,* einer Unterfamilie der Familie der Korbblütler (*Asteraceae*)*.* Zu den *Carduoideae* zählen beispielsweise Ringdisteln (*Carduus*), Golddisteln oder Eberwurzen (*Carlina*), Färberdisteln (*Carthamus*)*,* Kratzdisteln (*Cirsium*)*,* Kugeldisteln (*Echinops*)*,* Milchfleckdisteln (*Galactites*)*,* darunter die Milchfleckdistel (*Galactites tomentosus*)*,* Eselsdisteln (*Onopordum*)*,* Elfenbeindisteln (*Ptilostemon*)*,* darunter die Elfenbeindistel (*Ptilostemon afer*)*,* Mariendisteln (*Silybum*) und Gänsedisteln (*Sonchus*)*.*

Bei der aus Disteln (*Carduoideae*) erhältlichen Komponente der vorliegenden Erfindung kann es sich um einen Extrakt aus Disteln (*Carduoideae*) oder Pflanzenteilen von Disteln (*Carduoideae*) oder um ein Distel (*Carduoideae*)-Öl handeln. Distel (*Carduoideae*)-Öl ist ein aus Disteln (*Carduoideae*) oder Teilen davon gewonnenes Öl. Verfahren zur Herstellung von Ölen aus Pflanzen oder Pflanzenteilen sind dem Fachmann bekannt. Das Öl kann sowohl aus frischen als auch aus getrockneten Pflanzen oder Pflanzenteilen gewonnen werden. Bei dem Extrakt kann es sich um einen anorganischen oder organischen Extrakt handeln, es können als Extraktionsmittel also sowohl anorganische Lösungsmittel, wie Wasser, Säuren oder Basen, als auch organische Lösungsmittel, wie Alkohole, Terpene, Diethylether, pflanzliche Öle, chlorierte Kohlenwasserstoffe oder n-Hexan, eingesetzt werden. Bevorzugt handelt es sich um einen organischen Extrakt, mehr bevorzugt um einen organischen Extrakt mit einem pflanzlichen Öl als Extraktionsmittel. Bevorzugt handelt es sich um einen organischen Extrakt mit Olivenöl als Extraktionsmittel.

Überraschenderweise wurde festgestellt, dass die erfindungsgemäße Hautpflegezusammensetzung eine Reduktion von Hautfalten, insbesondere altersbedingten Hautfalten, bewirkt. Neu gebildete Falten verschwinden bei Verwendung der erfindungsgemäßen Hautpflegezusammensetzung fast vollständig, die Anzahl von bereits länger bestehenden Hautfalten wird um 80 bis 90% reduziert.

Bevorzugt umfasst die erfindungsgemäße Hautpflegezusammensetzung zwischen 5 und 20 Gew.-% Collagen, mehr bevorzugt zwischen 5 und 15 Gew.-% Collagen, noch mehr bevorzugt zwischen 7 und 11 Gew.-% Collagen, jeweils bezogen auf das Gesamtgewicht der Zusammensetzung.

Bevorzugt umfasst die erfindungsgemäße Hautpflegezusammensetzung zwischen 10 und 25 Gew.-% der aus Strohblumen (*Helichrysum*) erhältlichen Komponente, mehr bevorzugt zwischen 10 und 20 Gew.-% der aus Strohblumen (*Helichrysum*) erhältlichen Komponente, noch mehr bevorzugt zwischen 14 und 17 Gew.-% der aus Strohblumen (*Helichrysum*) erhältlichen Komponente, jeweils bezogen auf das Gesamtgewicht der Zusammensetzung.

Bevorzugt umfasst die erfindungsgemäße Hautpflegezusammensetzung zwischen 2 und 15 Gew.-% der aus Schwarzkümmel (*Nigella*) erhältlichen Komponente, mehr bevorzugt zwischen 2 und 10 Gew.-% der aus Schwarzkümmel (*Nigella*) erhältlichen Komponente, noch mehr bevorzugt zwischen 5 und 8 Gew.-% der aus Schwarzkümmel (*Nigella*) erhältlichen Komponente, jeweils bezogen auf das Gesamtgewicht der Zusammensetzung.

Bevorzugt umfasst die erfindungsgemäße Hautpflegezusammensetzung zwischen 5 und 20 Gew.-% der aus Disteln (*Carduoideae*) erhältlichen Komponente, mehr bevorzugt zwischen 5 und 15 Gew.-% der aus Disteln (*Carduoideae*) erhältlichen Komponente, noch mehr bevorzugt zwischen 7 und 11 Gew.-% der aus Disteln (*Carduoideae*) erhältlichen Komponente, jeweils bezogen auf das Gesamtgewicht der Zusammensetzung.

In einer bevorzugten Ausführungsform umfasst die erfindungsgemäße Hautpflegezusammensetzung
(i) zwischen 5 und 20 Gew.-% Collagen, und/oder
(ii) zwischen 10 und 25 Gew.-% der aus Strohblumen (*Helichrysum*) erhältlichen Komponente, und/oder
(iii) zwischen 2 und 15 Gew.-% der aus Schwarzkümmel (*Nigella*) erhältlichen Komponente, und/oder
(iv) zwischen 5 und 20 Gew.-% der aus Disteln (*Carduoideae*) erhältlichen Komponente,
jeweils bezogen auf das Gesamtgewicht der Zusammensetzung.

In einer weiteren bevorzugten Ausführungsform umfasst die erfindungsgemäße Hautpflegezusammensetzung
(i) zwischen 5 und 15 Gew.-% Collagen, und/oder
(ii) zwischen 10 und 20 Gew.-% der aus Strohblumen (*Helichrysum*) erhältlichen Komponente, und/oder
(iii) zwischen 2 und 10 Gew.-% der aus Schwarzkümmel (*Nigella*) erhältlichen Komponente, und/oder
(iv) zwischen 5 und 15 Gew.-% der aus Disteln (*Carduoideae*) erhältlichen Komponente, jeweils bezogen auf das Gesamtgewicht der Zusammensetzung.

In einer weiteren bevorzugten Ausführungsform umfasst die erfindungsgemäße Hautpflegezusammensetzung
(i) zwischen 7 und 11 Gew.-% Collagen, und/oder
(ii) zwischen 14 und 17 Gew.-% der aus Strohblumen (*Helichrysum*) erhältlichen Komponente, und/oder
(iii) zwischen 5 und 8 Gew.-% der aus Schwarzkümmel (*Nigella*) erhältlichen Komponente, und/oder
(iv) zwischen 7 und 11 Gew.-% der aus Disteln (*Carduoideae*) erhältlichen Komponente,
jeweils bezogen auf das Gesamtgewicht der Zusammensetzung.

Überraschenderweise wurde zudem festgestellt, dass die Wirkung der erfindungsgemäßen Hautpflegezusammensetzung auf die Reduktion von Hautfalten, insbesondere altersbedingten Hautfalten, verstärkt werden kann, wenn weitere Komponenten zu der Hautpflegezusammensetzung hinzugegeben werden.

In einer weiteren bevorzugten Ausführungsform umfasst die erfindungsgemäße Hautpflegezusammensetzung daher zusätzlich mindestens eine Komponente ausgewählt aus der Gruppe bestehend aus:
(v) Bienenwachs,
(vi) einer aus Avocado erhältlichen Komponente,
(vii) einer aus Hagebutten erhältlichen Komponente, und
(viii) einer aus Kokosnüssen erhältlichen Komponente.

Bienenwachs ist ein von Honigbienen abgesondertes Wachs. Es besteht aus Myricin (Anteil ca. 65 Gew-%), einem Gemisch von Estern langkettiger Alkohole (z.B. 1-Hentriacontanol) und Säuren, das von Palmitinsäuremyricylester C₁₅H₃₁-COO-C₃₀H₆₁ dominiert wird, daneben freier Cerotinsäure C₂₅H₅₁-COOH, Melissinsäure und ähnlichen Säuren (12 Gew.-%), gesättigten Kohlenwasserstoffen (ca. 14 Gew.-%), Alkoholen (ca. 1 Gew.-%) und anderen Stoffen (wie bienenartspezifischen Aromastoffen) (6 Gew.-%). Bienenwachs wird aufgrund seiner guten Hautverträglichkeit in der Kosmetik verwendet. Es erzeugt einen Schutzfilm auf der Haut, der vor Feuchtigkeitsverlust und Witterungseinflüssen schützt.

Bei der aus Avocado erhältlichen Komponente der vorliegenden Erfindung kann es sich um eine aus den Früchten des Avocadobaums (*Persea americana*) erhältliche Komponente handeln. Es kann sich um das aus dem Fruchtfleisch gewonnene Avocadoöl (PERSEA GRATISSIMA OIL, INCI) oder um das aus dem Avocadokern (Samen) gewonnene Fett (PERSEA GRATISSIMA BUTTER) handeln. Bevorzugt handelt es sich bei der aus Avocado erhältlichen Komponente der vorliegenden Erfindung um Avocadoöl. Avocadoöl ist ein Pflanzenöl, das aus den Früchten des Avocadobaums erhalten wird. Bei Avocadoöl handelt es sich um ein Fruchtfleischöl, welches bevorzugt durch Kaltpressung aus dem Fruchtfleisch gewonnen wird. Die Gewinnung kann auch enzymatisch unterstützt oder das Öl heißgepresst und mittels Lösungsmitteln extrahiert werden. Verfahren zur Herstellung von Avocadoöl sind dem Fachmann bekannt. Avocadoöl wird in der Kosmetik zur Herstellung von Hautpflegeprodukten verwendet.

Als Hagebutten bezeichnet man die ungiftigen Sammelnussfrüchte verschiedener Rosenarten, besonders der Hundsrose (*Rosa canina*)*.* Das Fruchtfleisch der im Spätherbst geernteten Früchte entsteht aus dem fleischigen Blütenboden, ist süßsauer und reich an Vitaminen, insbesondere Vitamin C (Ascorbinsäure), aber auch Vitamin A, B1 und B2. Bei der aus Hagebutten erhältlichen Komponente der vorliegenden Erfindung handelt es sich bevorzugt um Hagebuttenöl. Hagebuttenöl ist ein Pflanzenöl, das aus den Samen der Hagebutten gewonnen wird. Hagebuttenöl kann aus den in den Hagebutten enthaltenen Samen durch mechanisches Pressen oder über eine Extraktion gewonnen werden und wird anschließend bevorzugt raffiniert. Optional erfolgt eine Stabilisierung des Öls durch die Zugabe von Tocopherol (Vitamin E). Verfahren zur Herstellung von Hagebuttenöl sind dem Fachmann bekannt. Im Bereich der Kosmetik wird Hagebuttenöl wie andere Pflanzenöle in Salben und Cremes verwendet, vor allem in Nachtcremes.

Als Kokosnüsse werden die Früchte der Kokospalme (*Cocos nucifera*) bezeichnet. Bei der aus Kokosnüssen erhältlichen Komponente der vorliegenden Erfindung handelt es sich bevorzugt um Kokosöl, welches auch Kokosnussöl oder Kokosfett genannt wird. Kokosöl ist ein Pflanzenfett, welches aus Kopra, dem Nährgewebe der Kokosnuss, gewonnen wird. Kokosöl kann gewonnen werden, indem das Fruchtfleisch der Kokosnuss zerkleinert und getrocknet und anschließend in Ölmühlen ausgepresst wird. Optional kann das Kokosöl raffiniert und desodoriert werden. Verfahren zur Herstellung von Kokosöl sind dem Fachmann bekannt. Kokosöl (*Oleum cocos*) wird in der Kosmetik verwendet. Es zeigt eine feuchtigkeitsspendende Wirkung und hat einen kühlenden Effekt, dringt jedoch kaum in die Haut ein.

Die Hautpflegezusammensetzung kann eine, zwei, drei oder vier der zusätzlichen Komponenten (v) Bienenwachs, (vi) aus Avocado erhältliche Komponente, (vii) aus Hagebutten erhältliche Komponente und (viii) aus Kokosnüssen erhältliche Komponente umfassen.

In einer bevorzugten Ausführungsform der Erfindung umfasst die Hautpflegezusammensetzung:
(i) Collagen,
(ii) die aus Strohblumen (*Helichrysum*) erhältliche Komponente,
(iii) die aus Schwarzkümmel (*Nigella*) erhältliche Komponente,
(iv) die aus Disteln (*Carduoideae*) erhältliche Komponente,
(v) Bienenwachs,
(vi) eine aus Avocado erhältliche Komponente,
(vii) eine aus Hagebutten erhältliche Komponente, und
(viii) eine aus Kokosnüssen erhältliche Komponente.

In einer weiteren bevorzugten Ausführungsform der Erfindung umfasst die Hautpflegezusammensetzung
(i) zwischen 5 und 20 Gew.-% Collagen,
(ii) zwischen 10 und 25 Gew.-% der aus Strohblumen (*Helichrysum*) erhältlichen Komponente,
(iii) zwischen 2 und 15 Gew.-% der aus Schwarzkümmel (*Nigella*) erhältlichen Komponente,
(iv) zwischen 5 und 20 Gew.-% der aus Disteln (*Carduoideae*) erhältlichen Komponente,
(v) zwischen 20 und 35 Gew.-% Bienenwachs,
(vi) zwischen 0,5 und 5 Gew.-% einer aus Avocado erhältlichen Komponente,
(vii) zwischen 0,5 und 5 Gew.-% einer aus Hagebutten erhältlichen Komponente, und
(viii) zwischen 20 und 50 Gew.-% einer aus Kokosnüssen erhältlichen Komponente,
jeweils bezogen auf das Gesamtgewicht der Zusammensetzung.

In einer weiteren bevorzugten Ausführungsform der Erfindung umfasst die Hautpflegezusammensetzung
(i) zwischen 5 und 15 Gew.-% Collagen,
(ii) zwischen 10 und 20 Gew.-% der aus Strohblumen (*Helichrysum*) erhältlichen Komponente,
(iii) zwischen 2 und 10 Gew.-% der aus Schwarzkümmel (*Nigella*) erhältlichen Komponente,
(iv) zwischen 5 und 15 Gew.-% der aus Disteln (*Carduoideae*) erhältlichen Komponente,
(v) zwischen 20 und 30 Gew.-% Bienenwachs,
(vi) zwischen 0,5 und 3 Gew.-% einer aus Avocado erhältlichen Komponente,
(vii) zwischen 0,5 und 3 Gew.-% einer aus Hagebutten erhältlichen Komponente, und
(viii) zwischen 20 und 40 Gew.-% einer aus Kokosnüssen erhältlichen Komponente,
jeweils bezogen auf das Gesamtgewicht der Zusammensetzung.

In einer weiteren bevorzugten Ausführungsform der Erfindung umfasst die Hautpflegezusammensetzung
(i) zwischen 7 und 11 Gew.-% Collagen,
(ii) zwischen 14 und 17 Gew.-% der aus Strohblumen (*Helichrysum*) erhältlichen Komponente,
(iii) zwischen 5 und 8 Gew.-% der aus Schwarzkümmel (*Nigella*) erhältlichen Komponente,
(iv) zwischen 7 und 11 Gew.-% der aus Disteln (*Carduoideae*) erhältlichen Komponente,
(v) zwischen 23 und 27 Gew.-% Bienenwachs,
(vi) zwischen 1 und 2 Gew.-% einer aus Avocado erhältlichen Komponente,
(vii) zwischen 1 und 2 Gew.-% einer aus Hagebutten erhältlichen Komponente, und
(viii) zwischen 27 und 33 Gew.-% einer aus Kokosnüssen erhältlichen Komponente,
jeweils bezogen auf das Gesamtgewicht der Zusammensetzung.

Bevorzugt umfasst die erfindungsgemäße Hautpflegezusammensetzung zusätzlich einen Duftstoff, bevorzugt ausgewählt aus der Gruppe bestehend aus Bananenöl, Rosenöl und Lavendelöl.

Bevorzugt handelt es sich bei der aus Strohblumen *(Helichrysum)* erhältlichen Komponente der vorliegenden Erfindung um ein Strohblumen (*Helichrysum*)-Öl oder um einen organischen Extrakt aus Strohblumen (*Helichrysum*) oder Pflanzenteilen von Strohblumen (*Helichrysum*), mit einem pflanzlichen Öl als Extraktionsmittel. Strohblumen (*Helichrysum*)-Öl ist ein aus Strohblumen (*Helichrysum*) oder Teilen davon gewonnenes Öl. Das Öl kann sowohl aus frischen als auch aus getrockneten Strohblumen oder Teilen davon gewonnen werden. Das Strohblumen (*Helichrysum*)-Öl kann mittels dem Fachmann bekannten Verfahren gewonnen werden, beispielsweise durch Pressen (Kaltpressen, Heißpressen) der Pflanzen oder Pflanzenteile. Bevorzugt wird das Strohblumen (*Helichrysum*)-Öl durch Kaltpressen der Pflanzen oder Pflanzenteile gewonnen. Der organische Extrakt aus Strohblumen (*Helichrysum*) oder Pflanzenteilen von Strohblumen (*Helichrysum*)*,* mit einem pflanzlichen Öl als Extraktionsmittel, kann sowohl aus frischen als auch aus getrockneten Strohblumen oder Teilen davon gewonnen werden. Bevorzugt handelt es sich um einen organischen Extrakt aus Strohblumen (*Helichrysum*) oder Pflanzenteilen von Strohblumen (*Helichrysum*)*,* mit Olivenöl als Extraktionsmittel.

In einer weiteren bevorzugten Ausführungsform handelt es sich bei der aus Strohblumen (*Helichrysum*) erhältlichen Komponente der vorliegenden Erfindung um eine aus Sand-Strohblumen (*Helichrysum arenarium*) erhältliche Komponente. Die Sand-Strohblume (*Helichrysum arenarium*) ist eine Pflanzenart aus der Gattung der Strohblumen (*Helichrysum*) innerhalb der Familie der Korbblütler (*Asteraceae*)*.* Die Sand-Strohblume kommt von Südskandinavien über Mitteleuropa bis nach Südosteuropa, Osteuropa und weiter bis Zentralasien und zur Mongolei vor. Sie enthält ätherische Öle und Flavonoide und wird als Heilpflanze eingesetzt. So nennt der Ausschuss für pflanzliche Arzneimittel (HMPC) der Europäischen Arzneimittel-Agentur (EMA) beispielsweise in seiner Monographie *Helichrysum arenarium* die traditionelle Anwendung bei Verdauungsbeschwerden mit Völlegefühl und Blähungen. Bei der aus Sand-Strohblumen (*Helichrysum arenarium*) erhältlichen Komponente kann es sich um einen Extrakt aus Sand-Strohblumen (*Helichrysum arenarium*) oder Pflanzenteilen von Sand-Strohblumen (*Helichrysum arenarium*) oder um ein Sand-Strohblumen (*Helichrysum arenarium*)-Öl handeln. Sand-Strohblumen (*Helichrysum arenarium*)-Öl ist ein aus Sand-Strohblumen (*Helichrysum arenarium*) oder Teilen davon gewonnenes Öl. Das Öl kann sowohl aus frischen als auch aus getrockneten Sand-Strohblumen oder Teilen davon gewonnen werden. Verfahren zur Herstellung von Ölen aus Pflanzen oder Pflanzenteilen sind dem Fachmann bekannt. Bei dem Extrakt kann es sich um einen anorganischen oder organischen Extrakt handeln, es können als Extraktionsmittel also sowohl anorganische Lösungsmittel, wie Wasser, Säuren oder Basen, als auch organische Lösungsmittel, wie Alkohole, Terpene, Diethylether, pflanzliche Öle, chlorierte Kohlenwasserstoffe oder n-Hexan, eingesetzt werden. Bevorzugt handelt es sich um einen organischen Extrakt, mehr bevorzugt um einen organischen Extrakt mit einem pflanzlichen Öl als Extraktionsmittel. Bevorzugt handelt es sich um einen organischen Extrakt mit Olivenöl als Extraktionsmittel.

Besonders bevorzugt handelt es sich bei der aus Strohblumen *(Helichrysum)* erhältlichen Komponente der vorliegenden Erfindung um ein Sand-Strohblumen (*Helichrysum arenarium*)-Öl oder um einen organischen Extrakt aus Sand-Strohblumen (*Helichrysum arenarium*) oder Pflanzenteilen von Sand-Strohblumen (*Helichrysum arenarium*)*,* mit einem pflanzlichen Öl als Extraktionsmittel. Sand-Strohblumen (*Helichrysum arenarium*)-Öl ist ein aus Sand-Strohblumen (*Helichrysum arenarium*) oder Teilen davon gewonnenes Öl.

Das Öl kann sowohl aus frischen als auch aus getrockneten Sand-Strohblumen oder Teilen davon gewonnen werden. Das Sand-Strohblumen (*Helichrysum arenarium*)-Öl kann mittels dem Fachmann bekannten Verfahren gewonnen werden, beispielsweise durch Pressen (Kaltpressen, Heißpressen) der Pflanzen oder Pflanzenteile. Bevorzugt wird das Sand-Strohblumen (*Helichrysum arenarium*)-Öl durch Kaltpressen der Pflanzen oder Pflanzenteile gewonnen. Der organische Extrakt aus Sand-Strohblumen (*Helichrysum arenarium*) oder Pflanzenteilen von Sand-Strohblumen (*Helichrysum arenarium*)*,* mit einem pflanzlichen Öl als Extraktionsmittel, kann sowohl aus frischen als auch aus getrockneten Sand-Strohblumen oder Teilen davon gewonnen werden. Bevorzugt handelt es sich um einen organischen Extrakt aus Sand-Strohblumen (*Helichrysum arenarium*) oder Pflanzenteilen von Sand-Strohblumen (*Helichrysum arenarium*)*,* mit Olivenöl als Extraktionsmittel.

Bevorzugt handelt es sich bei der aus Schwarzkümmel (*Nigella*) erhältlichen Komponente der vorliegenden Erfindung um eine aus *Nigella sativa* erhältliche Komponente. *Nigella sativa* ist der botanische Name für den Echten Schwarzkümmel. Bei der aus *Nigella sativa* erhältlichen Komponente kann es sich um einen Extrakt aus *Nigella sativa* oder Pflanzenteilen von *Nigella sativa* oder um ein *Nigella sativa*-Öl handeln.

Bevorzugt handelt es sich bei der aus Schwarzkümmel (*Nigella*) erhältlichen Komponente um ein Schwarzkümmel (*Nigella*)-Öl, mehr bevorzugt um ein *Nigella sativa*-Öl. Schwarzkümmel (*Nigella*)-Öl ist ein aus Schwarzkümmeln *(Nigella)* oder Teilen davon gewonnenes Öl. *Nigella sativa*-Öl ist ein aus *Nigella sativa* oder Teilen davon gewonnenes Öl. Das Öl kann sowohl aus frischem als auch aus getrocknetem Schwarzkümmel oder Teilen davon gewonnen werden. Das Schwarzkümmel (*Nigella*)-Öl bzw. das *Nigella sativa*-Öl kann mittels dem Fachmann bekannten Verfahren gewonnen werden, beispielsweise durch Pressen (Kaltpressen, Heißpressen) der Pflanzen oder Pflanzenteile. Bevorzugt werden das Schwarzkümmel (*Nigella*)-Öl oder das *Nigella sativa*-Öl durch Kaltpressen der Pflanzen oder Pflanzenteile, bevorzugt durch Kaltpressen der Pflanzensamen, gewonnen. In der Naturheilkunde wird Schwarzkümmelöl bei Allergien, Neurodermitis, Psoriasis (Schuppenflechte), zur Regulierung des Immunsystems, gegen Asthma, in Begleitung von Chemotherapien zur Milderung der Nebenwirkungen, bei Verdauungsproblemen und Bluthochdruck verwendet, in der Tiermedizin unter anderem auch, um Zecken abzuschrecken.

Bei der aus Disteln (*Carduoideae*) erhältlichen Komponente der vorliegenden Erfindung kann es sich um eine aus Färberdisteln (*Carthamus*) erhältliche Komponente, mehr bevorzugt um eine aus *Carthamus tinctorius* erhältliche Komponente, handeln. Bevorzugt handelt es sich bei der aus Disteln (*Carduoideae*) erhältlichen Komponente um ein Distel (*Carduoideae*)-Öl, mehr bevorzugt um ein Färberdistel (*Carthamus*)-Öl, noch mehr bevorzugt um ein *Carthamus tinctorius*-Öl.

In einer bevorzugten Ausführungsform handelt es sich bei der aus Disteln (*Carduoideae*) erhältlichen Komponente der vorliegenden Erfindung um eine aus Mariendisteln (*Silybum*) erhältliche Komponente. Die Gattung der Mariendisteln (*Silybum*) umfasst zwei Arten: *Silybum eburneum* und die Mariendistel (*Silybum marianum*)*.* Besonders bevorzugt handelt es sich bei der aus Disteln (*Carduoideae*) erhältlichen Komponente der vorliegenden Erfindung um eine aus *Silybum marianum* (Mariendistel) erhältliche Komponente. Bei der aus Mariendisteln (*Silybum*) erhältlichen Komponente kann es sich um einen Extrakt aus Mariendisteln (*Silybum*) oder Pflanzenteilen von Mariendisteln (*Silybum*) oder um ein Mariendistel (*Silybum*)-Öl handeln. Mariendistel (*Silybum*)-Öl ist ein aus Pflanzen der Gattung der Mariendisteln (*Silybum*) oder Teilen davon gewonnenes Öl. Das Öl kann sowohl aus frischen als auch aus getrockneten Mariendisteln oder Teilen davon gewonnen werden. Bei dem Extrakt kann es sich um einen anorganischen oder organischen Extrakt handeln, es können als Extraktionsmittel also sowohl anorganische Lösungsmittel, wie Wasser, Säuren oder Basen, als auch organische Lösungsmittel, wie Alkohole, Terpene, Diethylether, pflanzliche Öle, chlorierte Kohlenwasserstoffe oder n-Hexan, eingesetzt werden. Bevorzugt handelt es sich um einen organischen Extrakt, mehr bevorzugt um einen organischen Extrakt mit einem pflanzlichen Öl als Extraktionsmittel. Bei der aus *Silybum marianum* (Mariendistel) erhältlichen Komponente kann es sich um einen Extrakt aus *Silybum marianum* (Mariendistel) oder Pflanzenteilen von *Silybum marianum* (Mariendistel) oder um ein *Silybum marianum*-Öl handeln. *Silybum marianum*-Öl ist ein aus *Silybum marianum* (Mariendistel) oder Teilen davon gewonnenes Öl. Das Öl kann sowohl aus frischen als auch aus getrockneten Mariendisteln (*Silybum marianum*) oder Teilen davon gewonnen werden. Bei dem Extrakt kann es sich um einen anorganischen oder organischen Extrakt handeln, es können als Extraktionsmittel also sowohl anorganische Lösungsmittel, wie Wasser, Säuren oder Basen, als auch organische Lösungsmittel, wie Alkohole, Terpene, Diethylether, pflanzliche Öle, chlorierte Kohlenwasserstoffe oder n-Hexan, eingesetzt werden. Bevorzugt handelt es sich um einen organischen Extrakt, mehr bevorzugt um einen organischen Extrakt mit einem pflanzlichen Öl als Extraktionsmittel.

In einer weiteren besonders bevorzugten Ausführungsform handelt es sich bei der aus Disteln (*Carduoideae*) erhältlichen Komponente um ein Distel (*Carduoideae*)-Öl. Distel (*Carduoideae*)-Öl ist ein aus Disteln (*Carduoideae*) oder Teilen davon gewonnenes Öl. Mehr bevorzugt handelt es sich bei der aus Disteln (*Carduoideae*) erhältlichen Komponente um ein Mariendistel (*Silybum*)-Öl. Mariendistel (*Silybum*)-Öl ist ein aus Pflanzen der Gattung der Mariendisteln (*Silybum*) oder Teilen davon gewonnenes Öl. Noch mehr bevorzugt handelt es sich bei der aus Disteln (*Carduoideae*) erhältlichen Komponente um ein *Silybum marianum*-Öl. *Silybum marianum*-Öl ist ein aus *Silybum marianum* oder Teilen davon gewonnenes Öl. Das Öl kann sowohl aus frischen als auch aus getrockneten Pflanzen oder Pflanzenteilen gewonnen werden. Das Öl kann mittels dem Fachmann bekannten Verfahren gewonnen werden, beispielsweise durch Pressen (Kaltpressen, Heißpressen) der Pflanzen oder Pflanzenteile. Bevorzugt wird das *Silybum marianum*-Öl durch Kaltpressen aus den Samen der Mariendistel (*Silybum marianum*) gewonnen.

In einer weiteren bevorzugten Ausführungsform umfasst die erfindungsgemäße Hautpflegezusammensetzung
(i) Collagen,
(ii) Sand-Strohblumen (*Helichrysum arenarium*)-Öl oder einen Sand-Strohblumen (*Helichrysum arenarium*)*-*Extrakt mit einem organischen Extraktionsmittel, bevorzugt mit einem pflanzlichen Öl als Extraktionsmittel,
(iii) Schwarzkümmel (*Nigella*)-Öl, bevorzugt *Nigella sativa*-Öl, und
(iv) Mariendistel (*Silybum*)-Öl, bevorzugt *Silybum marianum*-Öl.

Bevorzugt umfasst die Hautpflegezusammensetzung dieser Ausführungsform
(i) zwischen 5 und 20 Gew.-% Collagen,
(ii) zwischen 10 und 25 Gew.-% des Sand-Strohblumen (*Helichrysum arenarium*)-Öls oder des Sand-Strohblumen (*Helichrysum arenarium*)-Extrakts,
(iii) zwischen 2 und 15 Gew.-% Schwarzkümmel (*Nigella*)-Öl, bevorzugt zwischen 2 und 15 Gew.-% *Nigella sativa*-Öl, und
(iv) zwischen 5 und 20 Gew.-% Mariendistel (*Silybum*)-Öl, bevorzugt zwischen 5 und 20 Gew.-% *Silybum marianum*-Öl,
jeweils bezogen auf das Gesamtgewicht der Zusammensetzung.

Mehr bevorzugt umfasst die Hautpflegezusammensetzung dieser Ausführungsform
(i) zwischen 5 und 15 Gew.-% Collagen,
(ii) zwischen 10 und 20 Gew.-% des Sand-Strohblumen (*Helichrysum arenarium*)-Öls oder des Sand-Strohblumen (*Helichrysum arenarium*)-Extrakts,
(iii) zwischen 2 und 10 Gew.-% Schwarzkümmel (*Nigella*)-Öl, bevorzugt zwischen 2 und 10 Gew.-% *Nigella sativa*-Öl, und
(iv) zwischen 5 und 15 Gew.-% Mariendistel (*Silybum*)-Öl, bevorzugt zwischen 5 und 15 Gew.-% *Silybum marianum*-Öl,
jeweils bezogen auf das Gesamtgewicht der Zusammensetzung.

Noch mehr bevorzugt umfasst die Hautpflegezusammensetzung dieser Ausführungsform
(i) zwischen 7 und 11 Gew.-% Collagen,
(ii) zwischen 14 und 17 Gew.-% des Sand-Strohblumen (*Helichrysum arenarium*)-Öls oder des Sand-Strohblumen (*Helichrysum arenarium*)-Extrakts,
(iii) zwischen 5 und 8 Gew.-% Schwarzkümmel (*Nigella*)-Öl, bevorzugt zwischen 5 und 8 Gew.-% *Nigella sativa*-Öl, und
(iv) zwischen 7 und 11 Gew.-% Mariendistel (*Silybum*)-Öl, bevorzugt zwischen 7 und 11 Gew.-% *Silybum marianum*-Öl,
jeweils bezogen auf das Gesamtgewicht der Zusammensetzung.

In einer weiteren bevorzugten Ausführungsform umfasst die erfindungsgemäße Hautpflegezusammensetzung
(i) Collagen,
(ii) Sand-Strohblumen (*Helichrysum arenarium*)-Öl oder einen Sand-Strohblumen (*Helichrysum arenarium*)*-Extrakt* mit einem organischen Extraktionsmittel, bevorzugt mit einem pflanzlichen Öl als Extraktionsmittel,
(iii) Schwarzkümmel (*Nigella*)-Öl, bevorzugt *Nigella sativa*-Öl,
(iv) Mariendistel (*Silybum*)-Öl, bevorzugt *Silybum marianum*-Öl,
(v) Bienenwachs,
(vi) Avocadoöl,
(vii) Hagebuttenöl, und
(viii) Kokosöl.

Bevorzugt umfasst die Hautpflegezusammensetzung dieser Ausführungsform
(i) zwischen 5 und 20 Gew.-% Collagen,
(ii) zwischen 10 und 25 Gew.-% des Sand-Strohblumen (*Helichrysum arenarium*)-Öls oder des Sand-Strohblumen (*Helichrysum arenarium*)-Extrakts,
(iii) zwischen 2 und 15 Gew.-% Schwarzkümmel (*Nigella*)-Öl, bevorzugt zwischen 2 und 15 Gew.-% *Nigella sativa*-Öl,
(iv) zwischen 5 und 20 Gew.-% Mariendistel (*Silybum*)-Öl, bevorzugt zwischen 5 und 20 Gew.-% *Silybum marianum*-Öl,
(v) zwischen 20 und 35 Gew.-% Bienenwachs,
(vi) zwischen 0,5 und 5 Gew.-% Avocadoöl,
(vii) zwischen 0,5 und 5 Gew.-% Hagebuttenöl, und
(viii) zwischen 20 und 50 Gew.-% Kokosöl,
jeweils bezogen auf das Gesamtgewicht der Zusammensetzung.

Mehr bevorzugt umfasst die Hautpflegezusammensetzung dieser Ausführungsform
(i) zwischen 5 und 15 Gew.-% Collagen,
(ii) zwischen 10 und 20 Gew.-% des Sand-Strohblumen (*Helichrysum arenarium*)-Öls oder des Sand-Strohblumen (*Helichrysum arenarium*)-Extrakts,
(iii) zwischen 2 und 10 Gew.-% Schwarzkümmel (*Nigella*)-Öl, bevorzugt zwischen 2 und 10 Gew.-% *Nigella sativa*-Öl,
(iv) zwischen 5 und 15 Gew.-% Mariendistel (*Silybum*)-Öl, bevorzugt zwischen 5 und 15 Gew.-% *Silybum marianum*-Öl,
(v) zwischen 20 und 30 Gew.-% Bienenwachs,
(vi) zwischen 0,5 und 3 Gew.-% Avocadoöl,
(vii) zwischen 0,5 und 3 Gew.-% Hagebuttenöl, und
(viii) zwischen 20 und 40 Gew.-% Kokosöl,
jeweils bezogen auf das Gesamtgewicht der Zusammensetzung.

Noch mehr bevorzugt umfasst die Hautpflegezusammensetzung dieser Ausführungsform
(i) zwischen 7 und 11 Gew.-% Collagen,
(ii) zwischen 14 und 17 Gew.-% des Sand-Strohblumen (*Helichrysum arenarium*)-Öls oder des Sand-Strohblumen (*Helichrysum arenarium*)-Extrakts,
(iii) zwischen 5 und 8 Gew.-% Schwarzkümmel (*Nigella*)-Öl, bevorzugt zwischen 5 und 8 Gew.-% *Nigella sativa*-Öl,
(iv) zwischen 7 und 11 Gew.-% Mariendistel (*Silybum*)-Öl, bevorzugt zwischen 7 und 11 Gew.-% *Silybum marianum*-Öl,
(v) zwischen 23 und 27 Gew.-% Bienenwachs,
(vi) zwischen 1 und 2 Gew.-% Avocadoöl,
(vii) zwischen 1 und 2 Gew.-% Hagebuttenöl, und
(viii) zwischen 27 und 33 Gew.-% Kokosöl,
jeweils bezogen auf das Gesamtgewicht der Zusammensetzung.

Die vorliegende Erfindung betrifft weiterhin ein Verfahren zur Herstellung der erfindungsgemäßen Hautpflegezusammensetzung, umfassend:
- Vermischen von (i) Collagen, (ii) der aus Strohblumen (*Helichrysum*) erhältlichen Komponente, (iii) der aus Schwarzkümmel (*Nigella*) erhältlichen Komponente, (iv) der aus Disteln (*Carduoideae*) erhältlichen Komponente, sowie optional von (v) Bienenwachs, (vi) einer aus Avocado erhältlichen Komponente, (vii) einer aus Hagebutten erhältlichen Komponente, und/oder (viii) einer aus Kokosnüssen erhältlichen Komponente.

Bei dem erfindungsgemäßen Verfahren werden die in der Hautpflegezusammensetzung verwendeten Komponenten miteinander vermischt. Hierbei können die einzelnen Komponenten zunächst zusammengegeben und anschließend vermischt werden. Alternativ können die einzelnen Komponenten auch nach und nach zusammengegeben werden und jeweils vor Zugabe der nächsten Komponente miteinander vermischt werden. Die Reihenfolge der Zugabe ist hierbei beliebig.

Nach dem Vermischen wird jeweils eine homogene Mischung der einzelnen Komponenten erhalten. Bienenwachs weist einen Schmelzpunkt zwischen 62 und 65 °C auf. Wenn die Hautpflegezusammensetzung Bienenwachs umfasst, kann das Bienenwachs vor dem Vermischen mit den übrigen Komponenten durch Erwärmen, beispielsweise in einem Wasserbad, zum Schmelzen gebracht und verflüssigt werden, um das Vermischen zu erleichtern. Kokosöl weist einen Schmelzpunkt zwischen 23 und 26 °C auf und kann somit je nach Umgebungstemperatur fest oder flüssig sein. Wenn die Hautpflegezusammensetzung Kokosöl umfasst, kann dieses bei Bedarf vor dem Vermischen mit den übrigen Komponenten durch Erwärmen, beispielsweise in einem Wasserbad, verflüssigt werden, um das Vermischen zu erleichtern.

Es wurde festgestellt, dass die Hautpflegezusammensetzung reich an Vitaminen ist. Neben den Vitaminen C, A, B1, B2, B3, B6, B7, B9, E, und K enthält sie außerdem Magnesium, Selen, Zink, Chrom, Vanadium, Mangan, Calcium, Kalium, Natrium und Barium, Omega-3-Fettsäuren und Omega-6-Fettsäuren.

Überraschenderweise wurde festgestellt, dass die erfindungsgemäße Hautpflegezusammensetzung eine Reduktion von Hautfalten, insbesondere altersbedingten Hautfalten, bewirkt. Neu gebildete Falten verschwinden bei Verwendung der erfindungsgemäßen Hautpflegezusammensetzung fast vollständig, die Anzahl von bereits länger bestehenden Hautfalten wird um 80 bis 90% reduziert. Dieses Ergebnis ist bereits nach 7 bis 10 Tagen sichtbar (bei täglicher einmaliger Verwendung der Hautpflegezusammensetzung). Es wurde außerdem festgestellt, dass die erfindungsgemäße Hautpflegezusammensetzung eine Reduktion von Sommersprossen, Pigmentstörungen und/oder Altersflecken bewirkt sowie die Poren verkleinert. Auch diese Ergebnisse sind bereits nach 7 bis 10 Tagen sichtbar (bei täglicher einmaliger Verwendung der Hautpflegezusammensetzung). Die Hautpflegezusammensetzung ist für jeden Hauttyp geeignet, insbesondere für sehr empfindliche und allergische Hauttypen. Sie kann auch von Neurodermitis-Patienten verwendet werden. Es wurde zudem festgestellt, dass die Hautpflegezusammensetzung die Feuchtigkeit(sversorgung) der Haut verbessert, der Haut ein strahlendes Aussehen verleiht und die Frische der Haut steigert. Somit verbessert die Hautpflegezusammensetzung das Hautbild insgesamt.

Die vorliegende Erfindung betrifft daher die kosmetische Verwendung der erfindungsgemäßen Hautpflegezusammensetzung zum Erreichen mindestens eines Effektes ausgewählt aus der Gruppe bestehend aus:
i) Reduktion von Hautfalten,
ii) Reduktion von Sommersprossen, Pigmentstörungen und/oder Altersflecken auf der Haut,
iii) Verbesserung der Feuchtigkeit und/oder Feuchtigkeitsversorgung der Haut,
iv) Steigerung des Strahlens der Haut,
v) Steigerung der Frische der Haut, und
vi) Verfeinern des Hautbildes.

Besonders bevorzugt ist die kosmetische Verwendung der erfindungsgemäßen Hautpflegezusammensetzung zur Reduktion von Hautfalten.

Zur Erzielung der beschriebenen Effekte kann die Hautpflegezusammensetzung auf die Haut aufgetragen werden. Es können bei jeder Anwendung Mengen zwischen 1 und 5 mg, bevorzugt zwischen 2 und 3 mg, der Hautpflegezusammensetzung aufgetragen werden. Bevorzugt wird die Hautpflegezusammensetzung einmal oder zweimal täglich auf die Haut aufgetragen. Bevorzugt wird die Hautpflegezusammensetzung für mindestens 7 Tage, mehr bevorzugt für mindestens 10 Tage aufgetragen.

Überraschenderweise wurde außerdem festgestellt, dass die Hautpflegezusammensetzung neben den beschriebenen kosmetischen Effekten auch effizient zur Behandlung von Lippeninfektionen, wie Herpes, Bläschen oder Pickeln, eingesetzt werden kann. Außerdem kann sie effizient zur Behandlung von Insektenstichen, insbesondere Mückenstichen, eingesetzt werden und bietet zwischen 5 und 6 Stunden Schutz vor Juckreiz und beruhigt die Haut. Zudem wurde festgestellt, dass die Hautpflegezusammensetzung entzündete Haut beruhigt.

Die vorliegende Erfindung betrifft daher außerdem die erfindungsgemäße Hautpflegezusammensetzung zur Verwendung bei der Behandlung von Lippeninfektionen, insbesondere Herpes, und/oder Insektenstichen, insbesondere Mückenstichen.

Zur Erzielung der beschriebenen Effekte kann die Hautpflegezusammensetzung auf die von einem Insektenstich oder von einer Lippeninfektion betroffene Haut aufgetragen werden. Es können bei jeder Anwendung Mengen zwischen 1 und 5 mg, bevorzugt zwischen 2 und 3 mg, der Hautpflegezusammensetzung aufgetragen werden. Bevorzugt wird die Hautpflegezusammensetzung einmal oder zweimal täglich auf die betroffene Haut aufgetragen. Bevorzugt wird die Hautpflegezusammensetzung für mindestens 3 Tage, mehr bevorzugt für mindestens 5 Tage, noch mehr bevorzugt für mindestens 7 Tage aufgetragen.

Die Erfindung wird durch die nachfolgenden Beispiele näher erläutert:

### Beispiel 1: Herstellung der Hautpflegezusammensetzung

Es wurde eine Hautpflegezusammensetzung mit folgender Zusammensetzung hergestellt:

| **Komponente** | **Menge (mg)** | **Menge (Gew.-%)** |
|---|---|---|
| Collagen | 30 | 9,32 |
| Sand-Strohblumen-Extrakt (*Helichrysum arenarium*) | 50 | 15,53 |
| Schwarzkümmelöl (*Nigella sativa*) | 20 | 6,21 |
| Distelöl (*Silybum marianum*) | 30 | 9,32 |
| Bienenwachs | 80 | 24,84 |
| Avocadoöl | 6 | 1,86 |
| Hagebuttenöl | 6 | 1,86 |
| Kokosöl | 100 | 31,06 |
| Summe | 322 | 100,00 |

Das Collagen wurde durch das Auskochen von Schafsknochen gewonnen. Der Sand-Strohblumen-Extrakt (*Helichrysum arenarium*-Extrakt) wurde durch Extraktion mit Olivenöl aus den getrockneten Blüten von Sand-Strohblumen (*Helichrysum arenarium*) gewonnen. Das Schwarzkümmelöl wurde durch Kaltpressen aus den Samen des Echten Schwarzkümmel (*Nigella sativa*) erhalten. Das Distelöl wurde durch Kaltpressen aus den Samen der Mariendistel (*Silybum marianum*) erhalten. Das Bienenwachs wurde durch Kochen von Honigwaben gewonnen. Hierbei löste sich das Wachs komplett auf und trennte sich von Honig und Pollen. Nach dem Erkalten der Mischung wurde das Bienenwachs von diesen Bestandteilen abgetrennt. Bei dem Avocadoöl handelte es sich um kommerziell erhältliches Avocadoöl (reingepresstes Avocadoöl, ohne Zusatzstoffe; BIOTERRA ORGANIC SKINCARE). Bei dem Hagebuttenöl handelte es sich um kommerziell erhältliches Hagebuttenöl (kaltgepresst aus den Samen von Hagebutten; BIOTERRA ORGANIC SKINCARE). Bei dem Kokosöl handelte es sich um kommerziell erhältliches Kokosöl (Bio Kokosnussöl, nativ, kaltgepresst; dmBio).

Bienenwachs und Kokosöl wurden durch Erwärmen im Wasserbad geschmolzen. Anschließend wurden die einzelnen Komponenten zusammengegeben und durch Rühren vermischt. Die Hautpflegezusammensetzung wurde als viskose, ölige Masse erhalten.

### Beispiel 2: Verwendung der Hautpflegezusammensetzung

Die in Beispiel 1 erhaltene Hautpflegezusammensetzung wurde auf die gereinigte Gesichtshaut aufgetragen. Einmal täglich (abends) wurden jeweils 2 mg der Zusammensetzung aufgetragen und gleichmäßig über das ganze Gesicht verteilt. Bereits nach 7 Tagen der täglichen Anwendung der Hautpflegezusammensetzung ließ sich ein Rückgang von Hautfalten um 80 bis 90% beobachten. Neu gebildete Hautfalten verschwanden sogar nahezu vollständig.

Es wurde außerdem beobachtet, dass eine deutliche Reduktion von Pigmentflecken und Sommersprossen auf Gesichtspartien, auf die die Hautpflegezusammensetzung aufgetragen wurden, auftrat. Die Haut hatte ein strahlendes Aussehen und wies ein verbessertes Hautbild auf. Die Hautpflegezusammensetzung spendete der Haut zudem reichlich Feuchtigkeit, was zu dem strahlenden Aussehen beitrug.

### Beispiel 3: Hautpflegezusammensetzung zur Behandlung von Insektenstichen

Die in Beispiel 1 erhaltene Hautpflegezusammensetzung wurde auf die von einem Mückenstich betroffene Haut aufgetragen. Es wurden 2 mg der Zusammensetzung aufgetragen. Die Hautpflegezusammensetzung bot für 5 Stunden Schutz vor Juckreiz und beruhigte zudem die betroffene Haut.

### Beispiel 4: Hautpflegezusammensetzung zur Behandlung von Lippeninfektionen

Die in Beispiel 1 erhaltene Hautpflegezusammensetzung wurde auf eine von Herpes betroffene Lippenpartie aufgetragen. Es wurden einmal täglich (abends) 2 mg der Zusammensetzung aufgetragen. Bereits nach 3 Tagen wurde ein Rückgang der Herpesinfektion festgestellt.

## Patentansprüche

1. Hautpflegezusammensetzung umfassend
(i) Collagen,
(ii) eine aus Strohblumen (*Helichrysum*) erhältliche Komponente,
(iii) eine aus Schwarzkümmel (*Nigella*) erhältliche Komponente, und
(iv) eine aus Disteln (*Carduoideae*) erhältliche Komponente.

2. Hautpflegezusammensetzung nach Anspruch 1, wobei die Hautpflegezusammensetzung
(i) zwischen 5 und 20 Gew.-% Collagen, bevorzugt zwischen 5 und 15 Gew.-% Collagen, und/oder
(ii) zwischen 10 und 25 Gew.-% der aus Strohblumen (*Helichrysum*) erhältlichen Komponente, bevorzugt zwischen 10 und 20 Gew.-% der aus Strohblumen (*Helichrysum*) erhältlichen Komponente, und/oder
(iii) zwischen 2 und 15 Gew.-% der aus Schwarzkümmel (*Nigella*) erhältlichen Komponente, bevorzugt zwischen 2 und 10 Gew.-% der aus Schwarzkümmel (*Nigella*) erhältlichen Komponente, und/oder
(iv) zwischen 5 und 20 Gew.-% der aus Disteln (*Carduoideae*) erhältlichen Komponente, bevorzugt zwischen 5 und 15 Gew.-% der aus Disteln (*Carduoideae*) erhältlichen Komponente,
jeweils bezogen auf das Gesamtgewicht der Zusammensetzung, umfasst.

3. Hautpflegezusammensetzung nach einem der Ansprüche 1 oder 2, zusätzlich umfassend mindestens eine Komponente ausgewählt aus der Gruppe bestehend aus:
(v) Bienenwachs,
(vi) einer aus Avocado erhältlichen Komponente,
(vii) einer aus Hagebutten erhältlichen Komponente, und
(viii) einer aus Kokosnüssen erhältlichen Komponente.

4. Hautpflegezusammensetzung nach einem der Ansprüche 1 bis 3, wobei die Hautpflegezusammensetzung umfasst:
(i) Collagen,
(ii) die aus Strohblumen (*Helichrysum*) erhältliche Komponente,
(iii) die aus Schwarzkümmel (*Nigella*) erhältliche Komponente,
(iv) die aus Disteln (*Carduoideae*) erhältliche Komponente,
(v) Bienenwachs,
(vi) eine aus Avocado erhältliche Komponente,
(vii) eine aus Hagebutten erhältliche Komponente, und
(viii) eine aus Kokosnüssen erhältliche Komponente.

5. Hautpflegezusammensetzung nach einem der Ansprüche 1 bis 4, wobei die Hautpflegezusammensetzung
(i) zwischen 5 und 20 Gew.-% Collagen, bevorzugt zwischen 5 und 15 Gew.-% Collagen,
(ii) zwischen 10 und 25 Gew.-% der aus Strohblumen (*Helichrysum*) erhältlichen Komponente, bevorzugt zwischen 10 und 20 Gew.-% der aus Strohblumen (*Helichrysum*) erhältlichen Komponente,
(iii) zwischen 2 und 15 Gew.-% der aus Schwarzkümmel (*Nigella*) erhältlichen Komponente, bevorzugt zwischen 2 und 10 Gew.-% der aus Schwarzkümmel (*Nigella*) erhältlichen Komponente,
(iv) zwischen 5 und 20 Gew.-% der aus Disteln (*Carduoideae*) erhältlichen Komponente, bevorzugt zwischen 5 und 15 Gew.-% der aus Disteln (*Carduoideae*) erhältlichen Komponente,
(v) zwischen 20 und 35 Gew.-% Bienenwachs, bevorzugt zwischen 20 und 30 Gew.-% Bienenwachs,
(vi) zwischen 0,5 und 5 Gew.-% einer aus Avocado erhältlichen Komponente, bevorzugt zwischen 0,5 und 3 Gew.-% einer aus Avocado erhältlichen Komponente,
(vii) zwischen 0,5 und 5 Gew.-% einer aus Hagebutten erhältlichen Komponente, bevorzugt zwischen 0,5 und 3 Gew.-% einer aus Hagebutten erhältlichen Komponente, und
(viii) zwischen 20 und 50 Gew.-% einer aus Kokosnüssen erhältlichen Komponente, bevorzugt zwischen 20 und 40 Gew.-% einer aus Kokosnüssen erhältlichen Komponente,
jeweils bezogen auf das Gesamtgewicht der Zusammensetzung, umfasst.

6. Hautpflegezusammensetzung nach einem der Ansprüche 1 bis 5, wobei die Hautpflegezusammensetzung zusätzlich einen Duftstoff umfasst, bevorzugt ausgewählt aus der Gruppe bestehend aus Bananenöl, Rosenöl und Lavendelöl.

7. Hautpflegezusammensetzung nach einem der Ansprüche 1 bis 6, wobei es sich bei der aus Strohblumen (*Helichrysum*) erhältlichen Komponente um eine aus Sand-Strohblumen (*Helichrysum arenarium*) erhältliche Komponente handelt.

8. Hautpflegezusammensetzung nach einem der Ansprüche 1 bis 7, wobei es sich bei der aus Strohblumen (*Helichrysum*) erhältlichen Komponente um ein Strohblumen (*Helichrysum*)-Öl oder um einen organischen Extrakt aus Strohblumen (*Helichrysum*) oder Pflanzenteilen von Strohblumen (*Helichrysum*), mit einem pflanzlichen Öl als Extraktionsmittel, bevorzugt um ein Sand-Strohblumen (*Helichrysum arenarium*)-Öl oder um einen organischen Extrakt aus Sand-Strohblumen (*Helichrysum arenarium*) oder Pflanzenteilen von Sand-Strohblumen (*Helichrysum arenarium*), mit einem pflanzlichen Öl als Extraktionsmittel, handelt.

9. Hautpflegezusammensetzung nach einem der Ansprüche 1 bis 8, wobei es sich bei der aus Schwarzkümmel (*Nigella*) erhältlichen Komponente um eine aus *Nigella sativa* erhältliche Komponente handelt.

10. Hautpflegezusammensetzung nach einem der Ansprüche 1 bis 9, wobei es sich bei der aus Schwarzkümmel (*Nigella*) erhältlichen Komponente um ein Schwarzkümmel (*Nigella*)-Öl, bevorzugt um ein *Nigella sativa*-Öl, handelt.

11. Hautpflegezusammensetzung nach einem der Ansprüche 1 bis 10, wobei es sich bei der aus Disteln (*Carduoideae*) erhältlichen Komponente um eine aus Mariendisteln (*Silybum*) erhältliche Komponente, bevorzugt um eine aus *Silybum marianum* erhältliche Komponente, handelt.

12. Hautpflegezusammensetzung nach einem der Ansprüche 1 bis 11, wobei es sich bei der aus Disteln (*Carduoideae*) erhältlichen Komponente um ein Distel (*Carduoideae*)-Öl, bevorzugt um ein Mariendistel (*Silybum*)-Öl, mehr bevorzugt um ein *Silybum marianum*-Öl, handelt.

13. Verfahren zur Herstellung einer Hautpflegezusammensetzung nach einem der Ansprüche 1 bis 12, umfassend:
- Vermischen von (i) Collagen, (ii) der aus Strohblumen (*Helichrysum*) erhältlichen Komponente, (iii) der aus Schwarzkümmel (*Nigella*) erhältlichen Komponente, (iv) der aus Disteln (*Carduoideae*) erhältlichen Komponente, sowie optional von (v) Bienenwachs, (vi) einer aus Avocado erhältlichen Komponente, (vii) einer aus Hagebutten erhältlichen Komponente, und/oder (viii) einer aus Kokosnüssen erhältlichen Komponente.

14. Kosmetische Verwendung der Hautpflegezusammensetzung nach einem der Ansprüche 1 bis 12 zum Erreichen mindestens eines Effektes ausgewählt aus der Gruppe bestehend aus:
i) Reduktion von Hautfalten,
ii) Reduktion von Sommersprossen, Pigmentstörungen und/oder Altersflecken auf der Haut,
iii) Verbesserung der Feuchtigkeit und/oder Feuchtigkeitsversorgung der Haut,
iv) Steigerung des Strahlens der Haut,
v) Steigerung der Frische der Haut, und
vi) Verfeinern des Hautbildes.

15. Hautpflegezusammensetzung nach einem der Ansprüche 1 bis 12 zur Verwendung bei der Behandlung von Lippeninfektionen, insbesondere Herpes, und/oder Insektenstichen, insbesondere Mückenstichen.

## Geänderte Patentansprüche

### Geänderte Patentansprüche gemäss Regel 137(2) EPÜ.

1. Hautpflegezusammensetzung umfassend
(i) Collagen,
(ii) eine aus Strohblumen *(Helichrysum)* erhältliche Komponente,
(iii) eine aus Schwarzkümmel (*Nigella*) erhältliche Komponente, und
(iv) eine aus Disteln (*Carduoideae*) erhältliche Komponente.

2. Hautpflegezusammensetzung nach Anspruch 1, wobei die Hautpflegezusammensetzung
(i) zwischen 5 und 20 Gew.-% Collagen, bevorzugt zwischen 5 und 15 Gew.-% Collagen, und/oder
(ii) zwischen 10 und 25 Gew.-% der aus Strohblumen (*Helichrysum*) erhältlichen Komponente, bevorzugt zwischen 10 und 20 Gew.-% der aus Strohblumen (*Helichrysum*) erhältlichen Komponente, und/oder
(iii) zwischen 2 und 15 Gew.-% der aus Schwarzkümmel (*Nigella*) erhältlichen Komponente, bevorzugt zwischen 2 und 10 Gew.-% der aus Schwarzkümmel (*Nigella*) erhältlichen Komponente, und/oder
(iv) zwischen 5 und 20 Gew.-% der aus Disteln (*Carduoideae*) erhältlichen Komponente, bevorzugt zwischen 5 und 15 Gew.-% der aus Disteln (*Carduoideae*) erhältlichen Komponente,
jeweils bezogen auf das Gesamtgewicht der Zusammensetzung, umfasst.

3. Hautpflegezusammensetzung nach einem der Ansprüche 1 oder 2, zusätzlich umfassend mindestens eine Komponente ausgewählt aus der Gruppe bestehend aus:
(v) Bienenwachs,
(vi) einer aus Avocado erhältlichen Komponente,
(vii) einer aus Hagebutten erhältlichen Komponente, und
(viii) einer aus Kokosnüssen erhältlichen Komponente.

4. Hautpflegezusammensetzung nach einem der Ansprüche 1 bis 3, wobei die Hautpflegezusammensetzung umfasst:
(i) Collagen,
(ii) die aus Strohblumen (*Helichrysum*) erhältliche Komponente,
(iii) die aus Schwarzkümmel (*Nigella*) erhältliche Komponente,
(iv) die aus Disteln (*Carduoideae*) erhältliche Komponente,
(v) Bienenwachs,
(vi) eine aus Avocado erhältliche Komponente,
(vii) eine aus Hagebutten erhältliche Komponente, und
(viii) eine aus Kokosnüssen erhältliche Komponente.

5. Hautpflegezusammensetzung nach einem der Ansprüche 1 bis 4, wobei die Hautpflegezusammensetzung
(i) zwischen 5 und 20 Gew.-% Collagen, bevorzugt zwischen 5 und 15 Gew.-% Collagen,
(ii) zwischen 10 und 25 Gew.-% der aus Strohblumen (*Helichrysum*) erhältlichen Komponente, bevorzugt zwischen 10 und 20 Gew.-% der aus Strohblumen (*Helichrysum*) erhältlichen Komponente,
(iii) zwischen 2 und 15 Gew.-% der aus Schwarzkümmel (*Nigella*) erhältlichen Komponente, bevorzugt zwischen 2 und 10 Gew.-% der aus Schwarzkümmel (*Nigella*) erhältlichen Komponente,
(iv) zwischen 5 und 20 Gew.-% der aus Disteln (*Carduoideae*) erhältlichen Komponente, bevorzugt zwischen 5 und 15 Gew.-% der aus Disteln (*Carduoideae*) erhältlichen Komponente,
(v) zwischen 20 und 35 Gew.-% Bienenwachs, bevorzugt zwischen 20 und 30 Gew.-% Bienenwachs,
(vi) zwischen 0,5 und 5 Gew.-% einer aus Avocado erhältlichen Komponente, bevorzugt zwischen 0,5 und 3 Gew.-% einer aus Avocado erhältlichen Komponente,
(vii) zwischen 0,5 und 5 Gew.-% einer aus Hagebutten erhältlichen Komponente, bevorzugt zwischen 0,5 und 3 Gew.-% einer aus Hagebutten erhältlichen Komponente, und
(viii) zwischen 20 und 50 Gew.-% einer aus Kokosnüssen erhältlichen Komponente, bevorzugt zwischen 20 und 40 Gew.-% einer aus Kokosnüssen erhältlichen Komponente,
jeweils bezogen auf das Gesamtgewicht der Zusammensetzung, umfasst.

6. Hautpflegezusammensetzung nach einem der Ansprüche 1 bis 5, wobei die Hautpflegezusammensetzung zusätzlich einen Duftstoff umfasst, bevorzugt ausgewählt aus der Gruppe bestehend aus Bananenöl, Rosenöl und Lavendelöl.

7. Hautpflegezusammensetzung nach einem der Ansprüche 1 bis 6, wobei es sich bei der aus Strohblumen (*Helichrysum*) erhältlichen Komponente um eine aus Sand-Strohblumen (*Helichrysum arenarium*) erhältliche Komponente handelt.

8. Hautpflegezusammensetzung nach einem der Ansprüche 1 bis 7, wobei es sich bei der aus Strohblumen (*Helichrysum*) erhältlichen Komponente um ein Strohblumen (*Helichrysum*)-Öl oder um einen organischen Extrakt aus Strohblumen (*Helichrysum*) oder Pflanzenteilen von Strohblumen (*Helichrysum*), mit einem pflanzlichen Öl als Extraktionsmittel, bevorzugt um ein Sand-Strohblumen (*Helichrysum arenarium*)-Öl oder um einen organischen Extrakt aus Sand-Strohblumen (*Helichrysum arenarium*) oder Pflanzenteilen von Sand-Strohblumen (*Helichrysum arenarium*)*,* mit einem pflanzlichen Öl als Extraktionsmittel, handelt.

9. Hautpflegezusammensetzung nach einem der Ansprüche 1 bis 8, wobei es sich bei der aus Schwarzkümmel (*Nigella*) erhältlichen Komponente um eine aus *Nigella sativa* erhältliche Komponente handelt.

10. Hautpflegezusammensetzung nach einem der Ansprüche 1 bis 9, wobei es sich bei der aus Schwarzkümmel (*Nigella*) erhältlichen Komponente um ein Schwarzkümmel (*Nigella*)-Öl, bevorzugt um ein *Nigella sativa*-Öl, handelt.

11. Hautpflegezusammensetzung nach einem der Ansprüche 1 bis 10, wobei es sich bei der aus Disteln (*Carduoideae*) erhältlichen Komponente um eine aus Mariendisteln (*Silybum*) erhältliche Komponente, bevorzugt um eine aus *Silybum marianum* erhältliche Komponente, handelt.

12. Hautpflegezusammensetzung nach einem der Ansprüche 1 bis 11, wobei es sich bei der aus Disteln (*Carduoideae*) erhältlichen Komponente um ein Distel (*Carduoideae*)-Öl, bevorzugt um ein Mariendistel (*Silybum*)-Öl, mehr bevorzugt um ein *Silybum marianum-*Öl, handelt.

13. Verfahren zur Herstellung einer Hautpflegezusammensetzung nach einem der Ansprüche 1 bis 12, umfassend:
- Vermischen von (i) Collagen, (ii) der aus Strohblumen (*Helichrysum*) erhältlichen Komponente, (iii) der aus Schwarzkümmel (*Nigella*) erhältlichen Komponente, (iv) der aus Disteln (*Carduoideae*) erhältlichen Komponente, sowie optional von (v) Bienenwachs, (vi) einer aus Avocado erhältlichen Komponente, (vii) einer aus Hagebutten erhältlichen Komponente, und/oder (viii) einer aus Kokosnüssen erhältlichen Komponente.

14. Kosmetische Verwendung der Hautpflegezusammensetzung nach einem der Ansprüche 1 bis 12 zum Erreichen mindestens eines Effektes ausgewählt aus der Gruppe bestehend aus:
i) Reduktion von Hautfalten,
ii) Reduktion von Sommersprossen, Pigmentstörungen und/oder Altersflecken auf der Haut, und
iii) Verbesserung der Feuchtigkeit und/oder Feuchtigkeitsversorgung der Haut.

15. Hautpflegezusammensetzung nach einem der Ansprüche 1 bis 12 zur Verwendung bei der Behandlung von Lippeninfektionen, insbesondere Herpes, und/oder Insektenstichen, insbesondere Mückenstichen.
